# EUROPEAN PATENT SPECIFICATION

(11) **EP 1 829 493 B1**
(45) Date of publication and mention of the grant of the patent: **14.09.2016**
(21) Application number: 05819476.2
(22) Date of filing: 22.12.2005
(51) Int. Cl.: A61B 18/08, A61B 17/00

(54) **HEAT GENERATING TREATMENT APPARATUS**
WÄRMEERZEUGENDES BEHANDLUNGSGERÄT
APPAREIL DE TRAITEMENT GENERANT DE LA CHALEUR

(30) Priority: 24.12.2004 JP 2004374972
(43) Date of publication of application: 05.09.2007
(73) Proprietor: Olympus Corporation, Tokyo 192-8507 (JP)
(72) Inventor: Miura, Keisuke c/o Olympus Medical Systems Corp., Shibuya-ku Tokyo 151-0072 (JP)
(74) Representative: von Hellfeld, Axel
(86) International application number: PCT/JP2005/023657
(87) International publication number: WO 2006/068243

(56) References cited:
- EP-A- 1 582 165
- WO-A-96/02180
- WO-A1-96/00528
- WO-A1-97/20510
- JP-A- 62 133 947
- JP-A- 2003 093 402
- US-A- 5 643 257
- US-A- 5 755 715
- US-A- 5 807 269
- US-A1- 2002 082 593
- US-B1- 6 267 758
- US-B1- 6 383 183
- US-B1- 6 464 696
- US-B1- 6 464 696

## Description

### Technical Field

The present invention relates to a heat treatment device, and more particularly, relates to a heat treatment device that performs a treatment by applying heat to a living organism.

### Background Art

In general, heat treatment devices are used in surgery or in internal medicine to perform a treatment such as dissection, coagulation, blood stanching of an affected part. The heat treatment devices include a treatment portion which has heating means for heating the affected part in the treatment portion. The treatment such as dissection, coagulation, blood stanching, or the like is performed by applying the heat generated by the heating means to the affected part.

With respect to such heat treatment device, for example, in Japanese Examined Patent Application Publication No. 53-9031, a heat treatment device that includes a treatment portion having a plurality of divided heater segments as heating means has been disclosed.

The treatment portion is configured to treat an affected part by applying a heat generated by the plurality of heater segments which are set by a same temperature setting to the affected part.

However, in the conventional heat treatment devices, the output control is performed by the constant temperature control. Accordingly, if the heating temperature is set to be relatively high, the coagulation of the living organism is not sufficiently performed but the dissection is quickly performed. On the other hand, if the heat generation temperature is set to be relatively low, the coagulation of the living organism is sufficiently performed but the dissection is relatively slowly performed. That is, in the conventional heat treatment devices, because the treatment is performed by the constant temperature control, it is difficult to quickly perform the dissection in the state that the living organism is sufficiently coagulated. The present invention has been made in view of the above-described problems, and an object of the present invention is to provide a heat treatment device that can perform an output control other than the constant temperature control to expand the range of options in treatment, for example, a heat treatment device that can improve an organism coagulation ability and can reduce an organism dissection time.

Further relevant background art is disclosed in the following documents: US 6 464 696 B1 (OYAMA MASAHIDE [JP] ET AL); US 5 755 715 A (STERN ROGER A [US] ET AL); EP 1 582 165 A (OLYMPUS CORP [JP]); US 5 643 257 A (COHEN DONALD [US] ET AL); US 5 807 269 A (QUINN MICHAEL D [US] ET AL) and WO 96/02180 A (HEMOSTATIX CORP [US]).

### Disclosure of Invention

### Means for Solving the Problem

A heat treatment device according to claim 1 is provided.

The invention is defined in independent claim 1, further preferred embodiments are defined in the dependent claims. Examples and embodiments disclosed in the description which do not fall under the scope of the claims are of exemplary nature only.

### Brief Description of the Drawings

Fig. 1 is a device configurational view illustrating an overall configuration of a heat treatment device according to a first embodiment.
Fig. 2 is an elevational view illustrating a device body viewed from a front panel side of the device body.
Fig. 3 is an appearance view illustrating a back panel of the device body.
Fig. 4 is an explanatory view illustrating a coagulation dissection forceps.
Fig. 5 is a schematic perspective view illustrating a heat treatment portion of a coagulation dissection forceps viewed from a side surface in a horizontal direction.
Fig. 6 is a schematic perspective view of the heat treatment portion of Fig. 5 viewed from an upper surface in a vertical direction.
Fig. 7 is a cross-sectional view of a heat treatment portion of a coagulation dissection forceps viewed from an upper surface in a vertical direction.
Fig. 8 is a cross-sectional view of a treatment portion of a coagulation dissection forceps viewed from a side surface in a horizontal direction.
Fig. 9 is a circuit block diagram of a heat treatment device.
Fig. 10 is an indicator chart illustrating types of output sequences.
Fig. 11 is an indicator chart illustrating an exemplary setting of an output sequence.
Fig. 12 is an indicator chart illustrating a relationship between control contents and set values.
Fig. 13 is an indicator chart illustrating control switching conditions.
Fig. 14 is a graph illustrating temperature variation of a heating element and variation of power applied to the heating element at a time of an output.
Fig. 15 is a flowchart for explaining an operation of a heat treatment device.
Fig. 16 is a flowchart following Fig. 15.
Fig. 17 is a flowchart following Fig. 15.
Fig. 18 is a flowchart following Fig. 17.
Fig. 19 is a flowchart following Fig. 15.
Fig. 20 is a graph illustrating temperature variation of a heating element and variation of power applied to the heating element in a case that a time timer functions.
Fig. 21 is a circuit block diagram of a heat treatment device according to a second embodiment.
Fig. 22 is a graph illustrating a state of a control set value and an operation result, and a set voltage value and an output voltage value.
Fig. 23 is a comparative explanatory diagram with Fig. 22 illustrating a prominent state of an output voltage due to a difference between a control set value and an operation result.
Fig. 24 is an elevational view illustrating a device body viewed from a front panel side of the device body according to a third embodiment.
Fig. 25 is an indicator chart illustrating output control combinations in a coagulation dissection setting of a heat treatment device and output control combinations in a coagulation setting of a heat treatment device.

### Best Mode for Carrying Out the Invention

Embodiments of the present invention will be described below referring to the drawings.

### (First Embodiment)

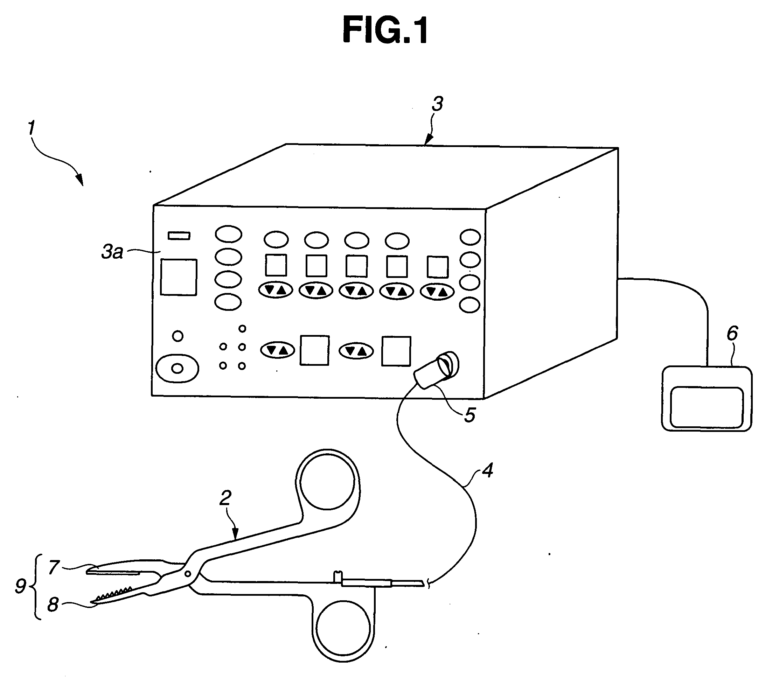

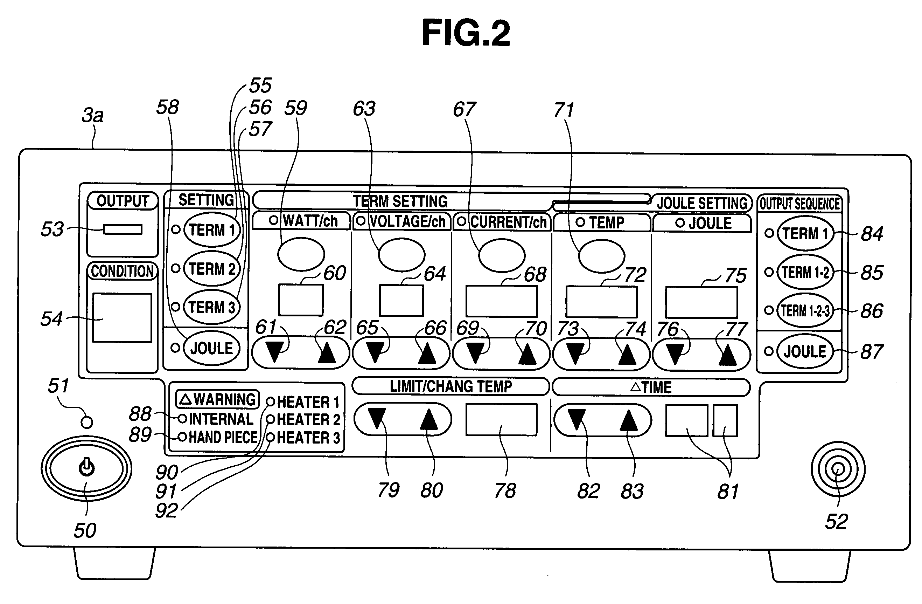

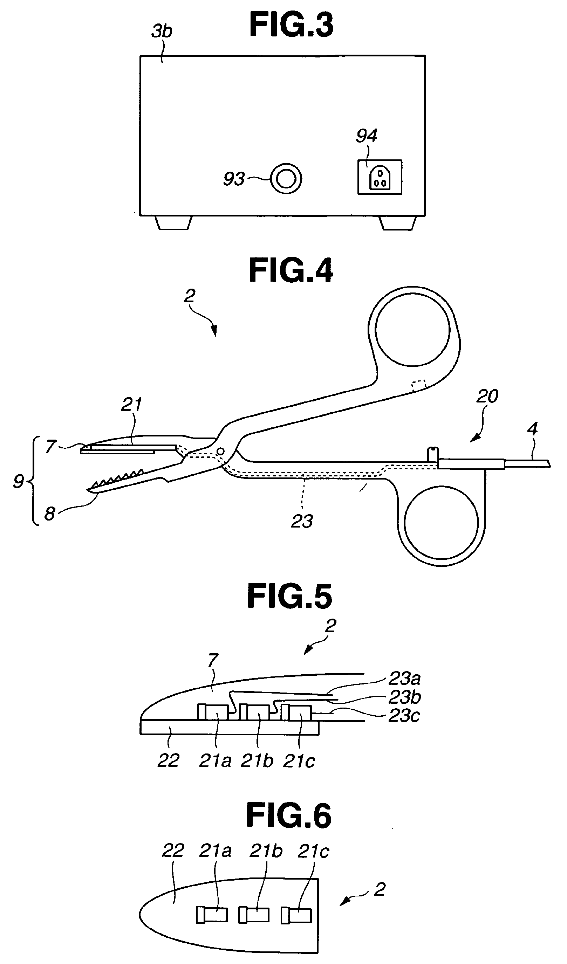

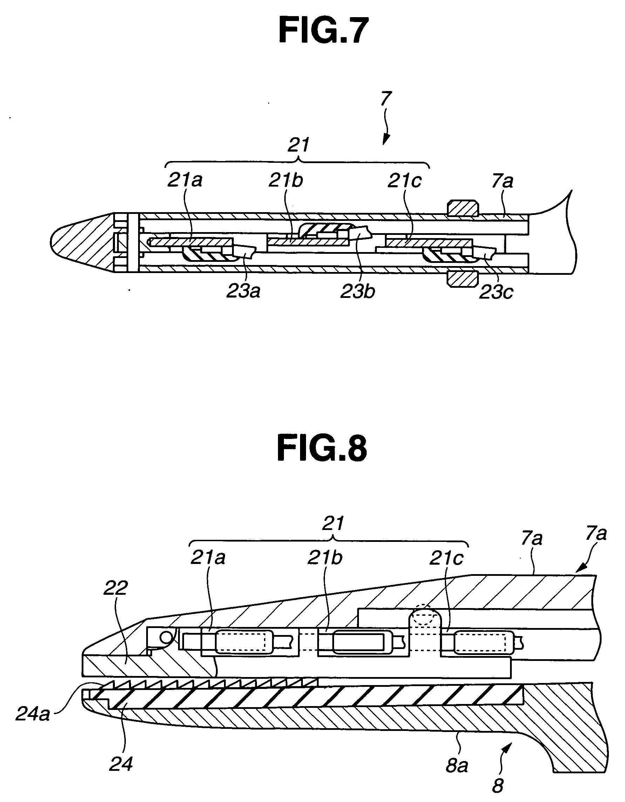

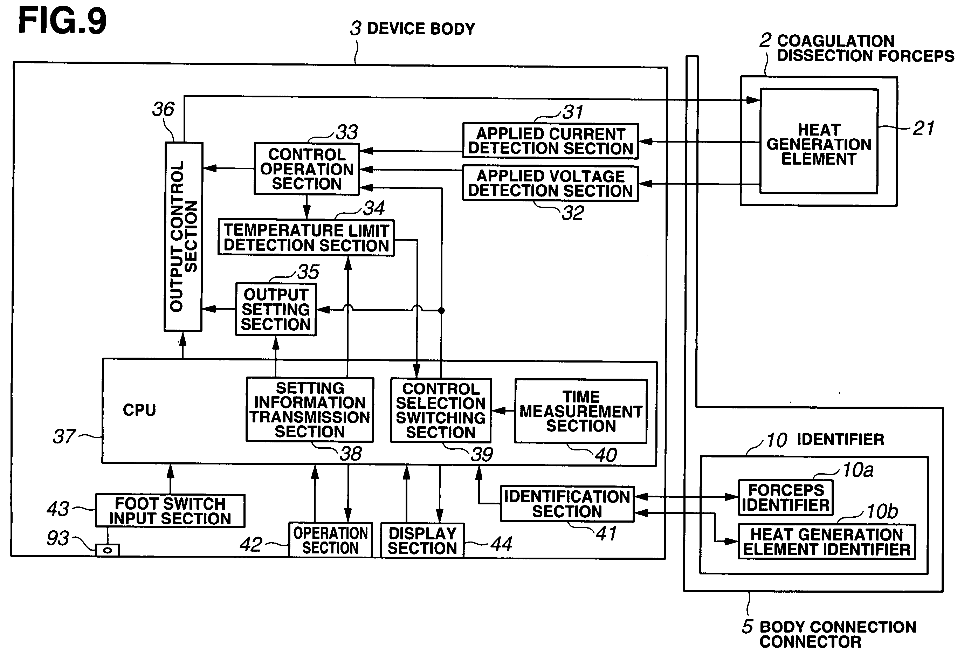

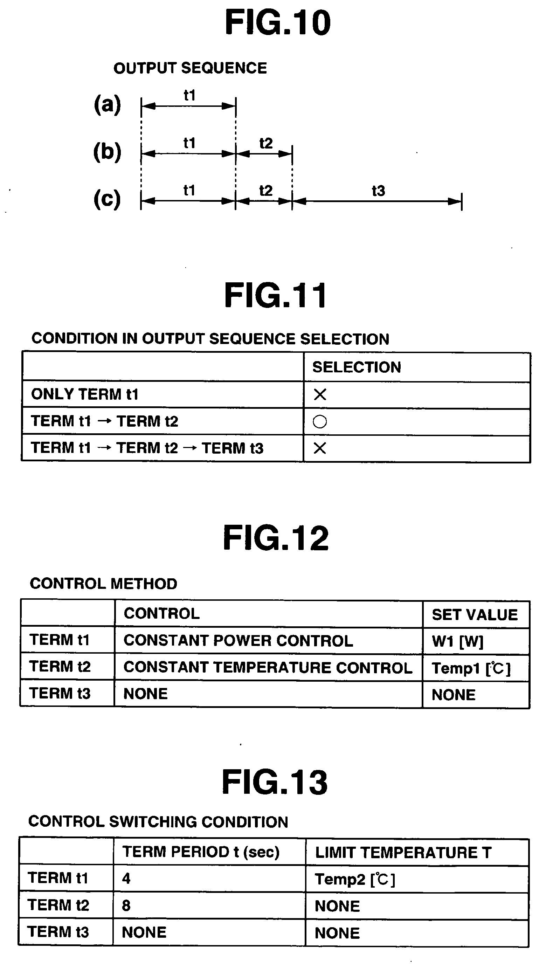

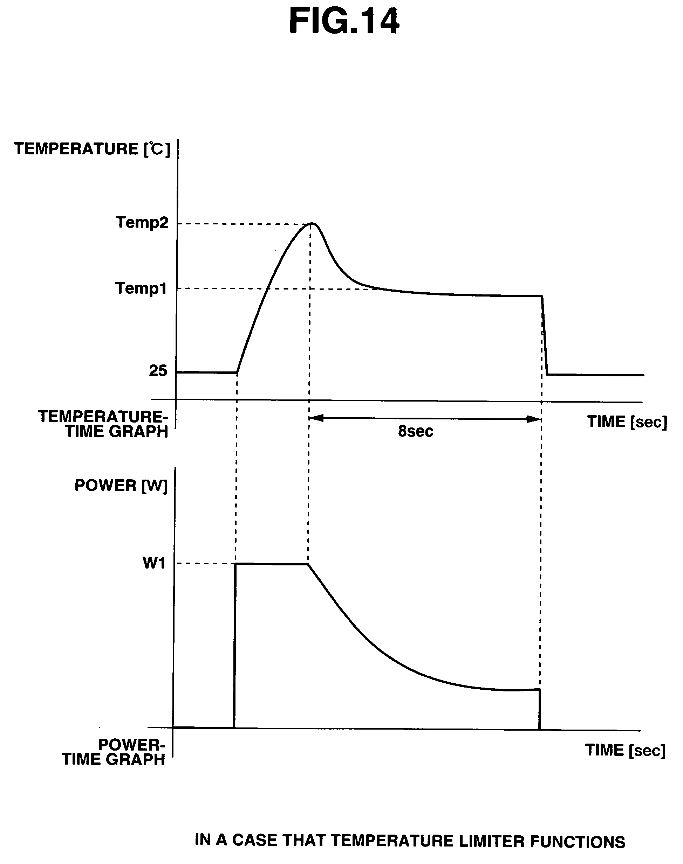

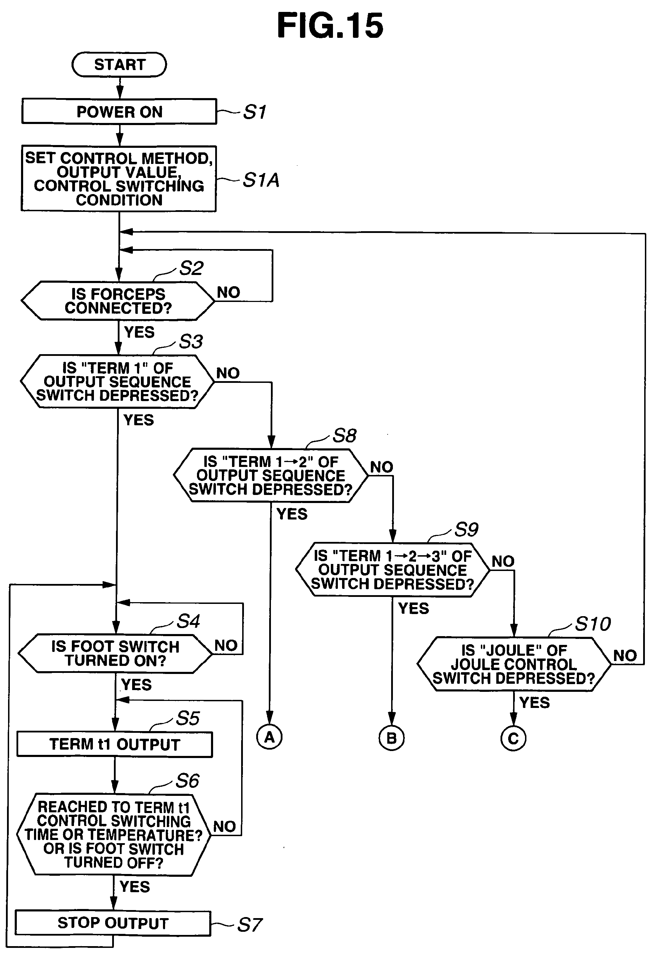

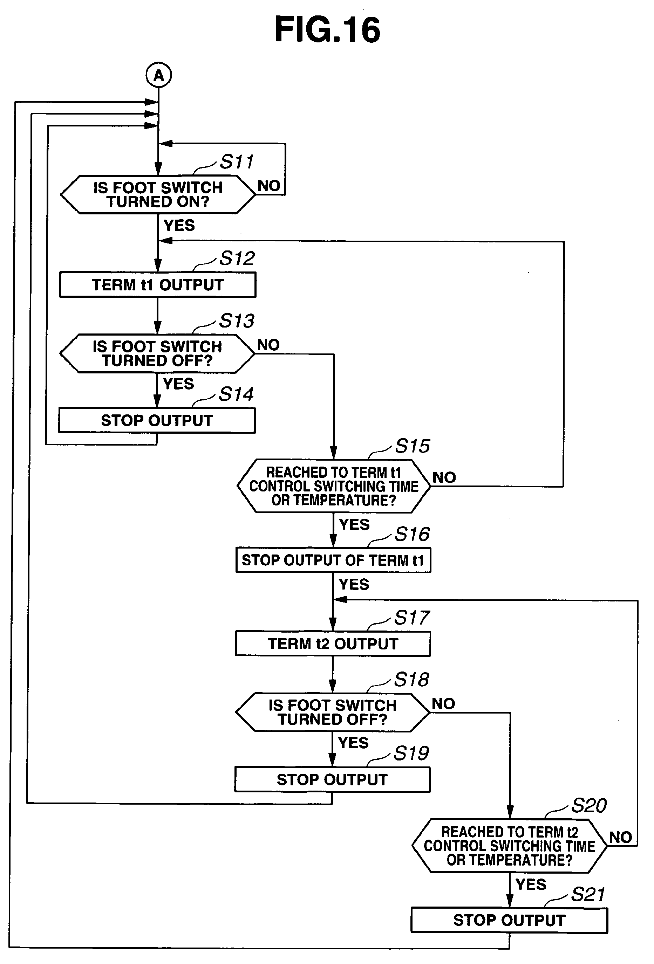

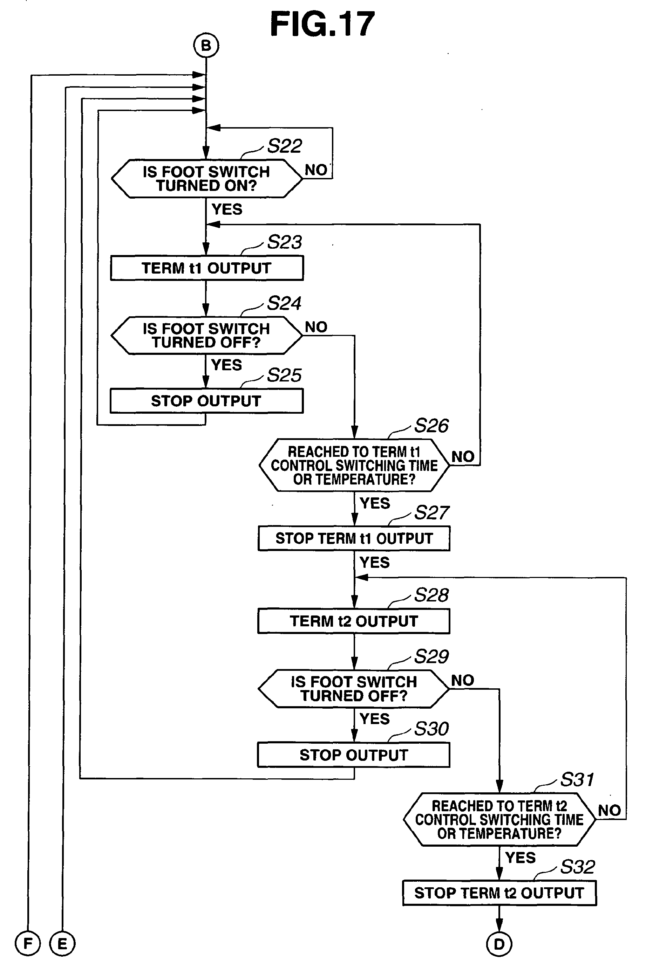

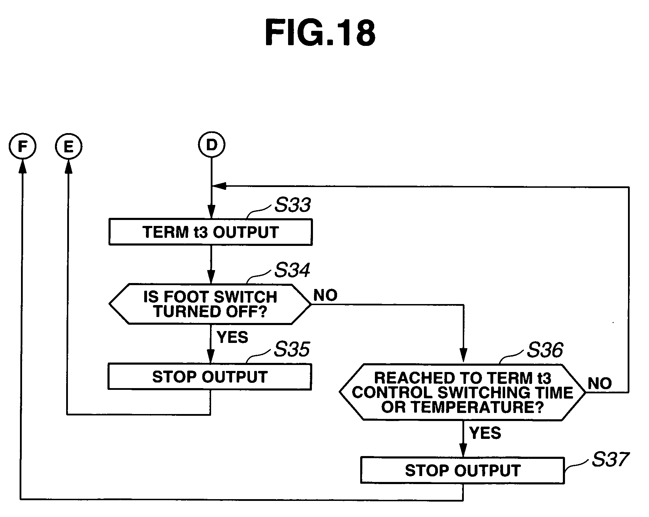

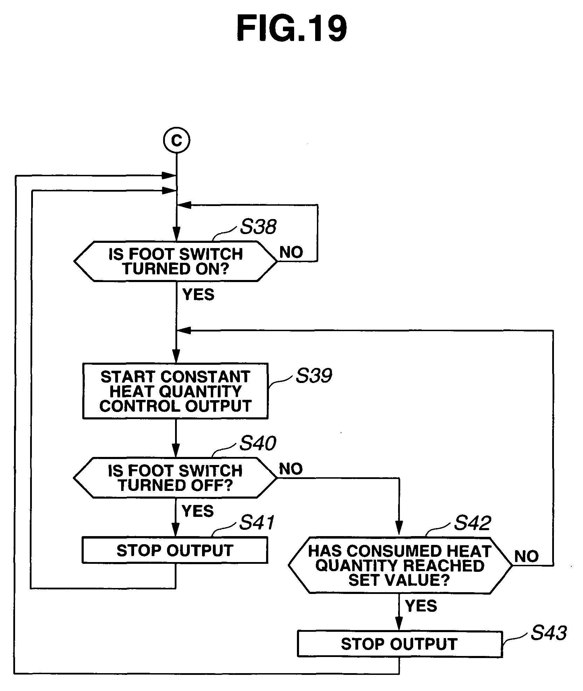

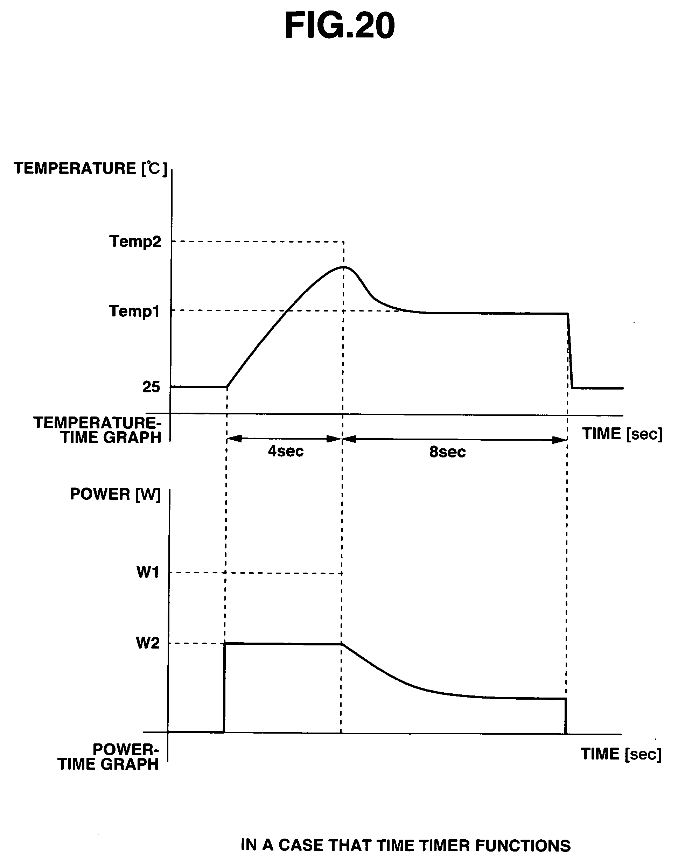

Figs. 1 to 20 relate to a first embodiment of the present invention. Fig. 1 is a device configurational view illustrating an overall configuration of a heat treatment device. Fig. 2 is an elevational view illustrating a device body viewed from a front panel side of the device body. Fig. 3 is an appearance view illustrating a back panel of the device body. Fig. 4 is an explanatory view illustrating a coagulation dissection forceps. Fig. 5 is a schematic perspective view illustrating a heat treatment portion of a coagulation dissection forceps viewed from a side surface in a horizontal direction. Fig. 6 is a schematic perspective view of the heat treatment portion of Fig. 5 viewed from an upper surface in a vertical direction. Fig. 7 is a cross-sectional view of a heat treatment portion of a coagulation dissection forceps viewed from an upper surface in a vertical direction. Fig. 8 is a cross-sectional view of a treatment portion of a coagulation dissection forceps viewed from a side surface in a horizontal direction. Fig. 9 is a circuit block diagram of a heat treatment device. Fig. 10 is an indicator chart illustrating types of output sequences. Fig. 11 is an indicator chart illustrating an exemplary setting of an output sequence. Fig. 12 is an indicator chart illustrating a relationship between control contents and set values. Fig. 13 is an indicator chart illustrating control switching conditions. Fig. 14 is a graph illustrating temperature variation of a heating element and variation of power applied to the heating element at a time of an output. Fig. 15 is a flowchart for explaining an operation of a heat treatment device. Figs. 16 and 17 are flowcharts following Fig. 15. Fig. 18 is a flowchart following Fig. 17. Fig. 19 is a flowchart following Fig. 15. Fig. 20 is a graph illustrating temperature variation of a heating element and variation of power applied to the heating element in a case that a time timer functions.

As shown in Fig. 1, a heat treatment device 1 according to the embodiment includes a coagulation dissection forceps 2 that functions as treatment means that includes a heating element, which is described below, and a device body 3 that detachably connects the coagulation dissection forceps 2 and performs a drive control by outputting power to the heating element of the coagulation dissection forceps 2. The device body 3 can be connected with a foot switch 6.

The coagulation dissection forceps 2 is configured to detachably connect a body connection connector 5 which is provided at a proximal end portion of an extended connection cable 4 to the device body 3. Further, the coagulation dissection forceps 2 includes a heat treatment portion 7 which has a plurality of built-in heating elements 21, and a treatment portion 9 which has an elastic reception portion 8 which can be separated with respect to the heat treatment portion 7. The treatment portion 9 is configured to grasp a living organism and perform a predetermined treatment.

The heat treatment portion 7 and the elastic reception portion 8 of the treatment portion 9 are configured to grasp the living organism. With a heat of the heat treatment portion 7 generated in response to power supply from the device body 3, the grasped living organism is supplied with the thermal energy and a coagulation dissection or a coagulation of the organism is performed.

The number of the heating elements 21 is varied depending on a type of a forceps selected according to a treatment purpose. Accordingly, an identifier 10 is included in the body connection connector 5. As shown in Fig. 9, the identifier 10 includes a forceps identifier 10a which indicates the type of the forceps and a heating element identifier 10b which has information about each heating element. The identifier 10 is, for example, an electrical resistance element, or a nonvolatile memory on which the identification information has previously been stored.

As shown in Figs. 2 and 3, the device body 3 includes a front panel 3a and a back panel 3b.

As shown in Fig. 2, the front panel 3a includes a connector reception portion 52 which can detachably connect the body connection connector 5 of the coagulation dissection forceps 2. The front panel 3a further includes a power switch 50 which turns on or off a power source and a LED 51 which lights when the power is on.

Further, the front panel 3a includes the following various switches and display LEDs which are used to perform settings, for example, a setting of a control content such as an energy supply to the heat treatment portion 7 of the coagulation dissection forceps 2 or a switching condition. The front panel 3 a includes a term 1 setting switch 55 which is used to transfer to a control content setting state of an output term 1, a term 2 setting switch 56 which is used to transfer to a control content setting state of an output term 2, a term 3 setting switch 57 which is used to transfer to a control content setting state of an output term 3, and a joule setting switch 58 which is used to transfer to a joule control value setting state.

Further, the front panel 3a includes a power setting switch 59 which is used to transfer to a power control value setting state, a power set value display LED 60 which displays a power set value, a power set value DOWN switch 61 and a power set value UP switch 62 which are used to change the power set value, a voltage setting switch 63 which is used to transfer to a voltage control value setting state, a voltage set value display LED 64 which displays a voltage set value, a voltage set value DOWN switch 65 and a voltage set value UP switch 66 which are used to change the voltage set value, a current setting switch 67 which is used to transfer to a current control value setting state, a current set value display LED 68 which displays a current set value, a current set value DOWN switch 69 and a current set value UP switch 70 which are used to change the current set value, a temperature setting switch 71 which is used to transfer to a temperature control value setting state, a temperature set value display LED 72 which displays a temperature set value, a temperature set value DOWN switch 73 and a temperature set value UP switch 74 which are used to change the temperature set value, a joule set value display LED 75 which displays a joule set value, and a joule set value DOWN switch 76 and a joule set value UP switch 77 which are used to change the joule set value. The joule set value display LED 75 which displays the joule set value, the joule set value DOWN switch 76, and the joule set value UP switch 77 constitute heat quantity setting means.

Further, the front panel 3a includes temperature setting means, that is, a limit temperature set value display LED 78 which displays a set value of a limit temperature to finish a term control or to transfer to a next term control, a limit temperature set value DOWN switch 79 and a limit temperature set value UP switch 80 which are used to change the limit temperature set value. Further, the front panel 3a includes time setting means, that is, a term control time setting display LED 81 which displays a time of each control term, that is, an output time, a term control time DOWN switch 82 and a term control time UP switch 83 which are used to change the term control time.

Further, the front panel 3 a includes a first output sequence switch 84 which instructs an output sequence of only a term 1, a second output sequence switch 85 which instructs to perform an output sequence of a term 2 subsequently to the term 1 output (term 1 → term 2), a third output sequence switch 86 which instructs to perform an output sequence of the term 2 and a term 3 subsequently to the term 1 output (term 1 → term 2 → term 3), and a joule control switch 87 which instructs to output by a joule control. These switches 84 to 87 and a setting information transmission section 38 constitute control selection instruction means.

Further, the front panel 3a includes a status display LED 54 which displays a status of the device body 3, an output display LED 53 which displays that a power is supplied to the heating element 21 of the coagulation dissection forceps 2, forceps error display LEDs 89 to 92 which light when there is an error in the coagulation dissection forceps 2, a power error display LED 88 which lights when there is an error in an internal circuit, and a buzzer (not shown) which sounds a warning sound.

Meanwhile, as shown in Fig. 3, the back panel 3b includes a foot switch connector reception portion 93 and a power inlet 94. The device body 3 can be connected with, for example, the coagulation dissection forceps 2 which includes four heating elements at the maximum.

As shown in Fig. 4, the coagulation dissection forceps 2 includes, as described above, the treatment portion 9 which has the heat treatment portion 7 and the elastic reception portion 8, and includes a handle portion 20 which is used to perform an open/close operation to grasp the living organism by the treatment portion 9. The connection cable 4 is configured to extend from the proximal end side of the handle portion 20.

As shown in Figs. 5 and 6, in the heat treatment portion 7 of the coagulation dissection forceps 2, the plurality of heating elements 21, for example, three identical heating elements 21 a, 21 b, and 21 c are coupled such that a heat can be transmitted to and provided to a heat conduction plate 22.

Next, a detailed configuration of the treatment portion 9 which includes the heat treatment portion 7 and the elastic reception portion 8 is described.

The heat treatment portion 7, as shown in Figs. 7 and 8, includes the heating elements 21 (21a to 21c) in a heat treatment portion body 7a. Here, the heating elements 21 which function as heating means may be, for example, laminated resistive elements formed on a ceramic board. To these heating elements 21 (21a to 21c), one ends of respective lead wires 23 (23a, 23b, 23c) for electric conduction are connected. The other ends of these lead wires 23 are inserted and disposed through the connection cable 4, and connected to connector terminals (not shown) of the body connection connector 5.

As described above, the heating elements 21 (21 a to 21 c) are coupled such that the heat can be transmitted to the heat conduction plate 22, and the heat generated in these heating elements 21 (21a to 21c) is transmitted to the heat conduction plate 22. In the configuration shown in Fig. 7, the heating elements 21 (heating portion) and the heat conduction plate 22 (treatment portion) are independently provided. However, the heating portion and the treatment portion may be integrally formed as a heating treatment portion. Further, the integrally formed heating treatment portion may be a heating resistive metal material (for example, a nichrome wire).

Meanwhile, as shown in Fig. 8, the elastic reception portion 8 includes, between the elastic reception portion 8 and the heat conductive plate 22 of the heat treatment portion 7, an elastic member 24 which has a sawtooth portion 24a which can grasp the living organism on the elastic reception portion body 8a. With a closing operation of the handle portion 20, the elastic reception portion 8 is closed with respect to the heat treatment portion 7, and the living organism is elastically grasped by the heat conductive plate 22 of the heat treatment portion 7 and the sawtooth portion 24a of the elastic reception portion 8. The living organism being sandwiched by the heat conductive plate 22 and the elastic member 24 is coagulated and dissected or coagulated by the heat of the heat conductive plate 22. The shapes of the heat treatment portion 7 and the elastic reception portion 8 which constitute the treatment portion 9 are not limited to the above-described shapes.

Now, with reference to a block diagram of Fig. 9, the device body 3 is described.

In the device body 3, an output of driving power for generating heat in the heating elements 21 is controlled by an output control section 36.

An applied current detection section 31 of the device body 3 functions as applied current detection means, and detects a current value applied to the heating elements 21. An applied voltage detection section 32 functions as applied voltage detection means, and detects a voltage value applied to the heating elements 21. A control operation section 33 which functions as operation means uses results from the applied current detection section 31 and the applied voltage detection section 32 to perform an operation necessary for a predetermined control which is instructed by a control selection switching section 39. Then, the control operation section 33 calculates a state of supply of energy, that is, power, to the heating elements 21, and a state of the heating elements 21, specifically, calculates a temperature (resistance value and consumed heat quantity). An output setting section 35 which functions as output setting means calculates a set value based on setting information of a setting information transmission section 38 and a control selection switching section 39. An output control section 36 which functions as output control means controls a current and/or a voltage such that the operation result of the control operation section 33 matches the set value of the output setting section 35.

The control operation section 33 can perform a temperature measurement using the variation of the resistance values of the heat generation elements 21 due to a temperature variation. That is, the control operation section 33, as a resistance value detection function, measures a value of the current running in the heat generation elements 21 which varies due to the variation in the resistance value in the heat generation elements 21, and a value of a voltage applied to the heat generation elements 21. Thus, the resistance value of the heat generation elements 21 is detected and a temperature of the heat generation elements 21 can be calculated based on the detected resistance value.

Here, an operation section 42 shown in Fig. 9 includes various switches such as the term 1 setting switch 55 provided on the front panel 3a, and various LEDs provided on the front panel 3 are represented as a display section 44.

Further, a temperature limit detection section 34 of the device body 3 functions as predetermined temperature detection means. If an operation result relating to a temperature of the control operation section 33 reaches a "temperature limit value" which is set by the setting information transmission section 38, the temperature limit detection section 34 sends a signal to "switch the controls" or "stop the control" to a control selection switching section 39. A time measurement section 40 functions as time measurement means. If an output time reaches a predetermined time arbitrarily set by the operation section 42, the time measurement section 40 sends information about passage of time to the control selection switching section 39. The setting information transmission section 38 sends the setting information inputted by the operation section 42 to the output setting section 35 and the temperature limit detection section 34.

As described above, the output setting section 35 calculates the set value based on the setting information of the setting information transmission section 38 and the control selection switching section 39. The control selection switching section 39 which functions as control selection means controls the output control section 36 which is control output means through the control operation section 33 and the output setting section 35 by sending the control selection information and the control switching information to the control operation section 33 and the output setting section 35. The output control section 36 controls, as described above, such that the operation result of the control operation section 33 matches the set value of the output setting section 35.

An identification section 41 of the device body 3 detects information in the identifier 10 which is provided to the body connection connector 5.

In the embodiment, the setting information transmission section 38, the control selection switching section 39, and the time measurement section 40 are provided in a CPU 37. However, these portions may be provided outside of the CPU 37.

To the device body 3, for example, a coagulation dissection forceps which has a maximum of four heating elements 21 can be connected. In such a case, corresponding to the respective heating elements 21, four channels of the applied current detection sections 31, the applied voltage detection sections 32, the control operation sections 33, temperature limit detection sections 34, the output setting sections 35, and the output control sections 36 perform their functions.

With reference to Figs. 10 and 11, functions of the heat treatment device 1 according to the embodiment are described.

The heat treatment device 1 can perform a "constant voltage control", a "constant current control", a "constant temperature control", a "constant power control", and a "constant heat quantity control (joule control)". The constant voltage control, the constant current control, and the constant power control provide the heating elements 21 with control contents of applications of a constant voltage, current, or power, respectively. In the constant temperature control, the applications of the current and the voltage are controlled such that a temperature of the heating elements 21 is to be constant. In the constant heat quantity control, if a heat quantity applied to the heating elements 21 from an output start reaches a certain set value (consumed heat quantity), the output is stopped.

Fig. 10 is a view illustrating types of output sequences. In the output sequences, there are three types of output sequences as shown in (a) to (c) of Fig. 10. In the heat treatment device 1, any of the output sequences can be selected. The drawing (a) shown in Fig. 10 is a control of only one step, in the drawing (b), the control contents can be switched up to two steps, and in the drawing (c), the controls can be switched up to three steps. T1 to t3 shown in Fig. 10 represent control terms. In each of the terms t1 to t3, any of the "constant voltage control", the "constant current control", and the "constant temperature control" can be performed. To the "constant heat quantity control", the above-described concept of the controls of the terms t1 to t3 is not applied. In the "constant heat quantity control", if the heat quantity applied to the heating elements 21 reaches the certain temperature, the output is stopped.

In the output sequences shown in Fig. 10, a condition for performing the stop of the output or the switch of the controls is a time or a predetermined temperature (limit temperature) in the heating elements 21. These values are to be set before the output.

With respect to the control switching by the heating element temperature (limit temperature), in the embodiment, the controls are switched even if only one of the plurality of heating elements exceeds the limit temperature. However, means which can select either of the "method of switching the control contents if only one of the plurality of heating elements exceeds the limit temperature" or a "method of switching the control contents if all of the plurality of heating elements exceed the limit temperature", that is, as condition selection means, for example, a limit temperature switching condition selection switch may be provided.

Figs. 11 to 13 illustrate an example of setting information according to the embodiment.

Fig. 11 illustrates a condition in an output sequence selection. The output sequence includes two terms, that is, a term t1 and a term t2, and a control is performed in the two terms. Fig. 12 illustrates control contents and set values in respective terms. The control content in the term t1 is defined by the constant power control to have a set value of W1 [W]. The control content in the term t2 is defined by the constant temperature control to have a set value of Temp 1 [°C]. Fig. 13 illustrates control switching conditions. Control switching conditions defined by time are four seconds in the term t1, and eight seconds in the term t2. A control switching condition defined by the limit temperature, that is, conditions for switching the controls from the term t1 to the term t2 (t1 → t2) is a set value of Temp 2 [°C]. The control selected in the term t2 is the constant temperature control. Accordingly, a switching condition with temperature does not exist in the term t2.

An operation of the thus configured heat treatment device 1 is described with reference to Figs. 9 to 19.

First, at step S1 shown in Fig, 15, a power is turned on. At step S 1A, as described below, control contents, output values, and control switching conditions of respective terms are set. In the following description, these settings are set according to contents shown in Figs. 11 to 13.

As shown in Fig. 11, settings of the output sequence is performed. There are two terms, that is, the term t1 and term t2 in the output sequence, and control contents, output values, and control switching conditions in at least the two terms are set. In a case that the setting of the term t1 is performed, a term 1 setting switch 55 shown in Fig. 2 is depressed to enter into a term t1 output control selection state. In the term t1, the constant power control is performed. Accordingly, a power setting switch 59 is depressed to enter into a power control set value input state. In the state, the power set value DOWN switch 61 and the power set value UP switch 62 are operated such that a display on the power set value display LED 60 is to be W1 [W].

Then, in the state, a condition to switch from the term t1 to the term t2 is set. The limit temperature to transfer to the term t2 is set to the Temp 2 [°C]. Accordingly, the limit temperature set value DOWN switch 79 and the limit temperature set value UP switch 80 are operated such that a display on the limit temperature set value display LED 78 is to be the Temp 2 [°C]. Then, because the time period of the term t1, that is, the output time is set to four seconds, the term control time DOWN switch 82 and the term control time UP switch 83 are operated such that a display on the term control time setting display LED 81 is to be four seconds.

Then, a setting of the term t2 is performed. In a case that the setting of the term t2 is performed, the term 2 setting switch 56 is depressed to enter into a term t2 output control selection state. In the term t2, the constant temperature control is performed. Accordingly, a temperature setting switch 71 is depressed to enter into a temperature control set value input state. In the state, the temperature set value DOWN switch 73 and the temperature set value UP switch 74 are operated such that a display on the temperature set value display LED is to be Temp 1 [°C]. Thus, the set temperature is set to the Temp 1 [°C]. Further, in the state, a condition (control switching condition) to finish the term t2 is set. In the examples shown in Figs. 11 and 12, the temperature control is selected in the term t2. Accordingly, as shown in Fig. 13, the setting of the limit temperature does not exist. Since the time period of the term t2, that is, the output time is set to eight seconds, the term control time DOWN switch 82 and the term control time UP switch 83 are operated such that the display on the term control time setting display LED 81 is to be eight seconds.

Next, at step S2, an output sequence switch, which is described below, functions only in a state that a forceps is connected. Accordingly, it is determined whether the forceps is connected or not. If the forceps is not connected, the process enters into a standby state.

Using the output sequence switch, an output sequence is determined as follows. In order to set the output sequence, as shown in (b) of Fig. 10, to two terms, that is, the term t1 and the term t2, at step S 8, a second output sequence switch 85 is depressed and a process enters into an output standby state. Then, the subsequent process proceeds to step S 11 of Fig. 16.

As shown in (a) of Fig. 10, to set the output sequence to only the term t1, at step S3, a first output sequence switch 84 is depressed to enter into an output standby state. The subsequent process proceeds to step S 4 of Fig. 15. Further, as shown in (c) of Fig. 10, to set the output sequence to the three terms, that is, the term t1, the term t2, and the term t3, at step S9, a third output sequence switch 86 is depressed to enter into an output standby state. The subsequent process proceeds to step S 22 of Fig. 17.

Now, with respect to a state of an output for performing a predetermined treatment is described with reference to Figs. 15 and 16.

After the organism to be treated is sandwiched in the treatment portion 9 of the coagulation dissection forceps 2, at step S11 of Fig. 16, the foot switch 6 that functions as output on/off means is turned on, and at step S12, an output of the term t1 is started. Then, at the steps S13 and S14, when the foot switch 6 is turned off, the output is stopped.

In response to the above-described start of the output of the term t1, as shown in a lower graph (power-time graph) of Fig. 14, first, the constant power control that is selected for the term t1 is performed in response to an application of the set value W1 [W]. Simultaneously with the output, as show in an upper graph (temperature - time graph) of Fig. 14, the temperature of the heating elements 21 is increased. At step S 15, as shown in Fig. 14, at a time point when the temperature reaches the limit temperature of the term t1 Temp 2 [°C], the process proceeds to step S16. Then the output of the term t1 is stopped, and the output control is switched to the constant temperature control of the term t2. At step S 17, the output is controlled such that the temperature of the heating elements is kept to be the Temp 1 [°C]. In such a case, the power may be applied such that the Temp 1 [°C] is kept. In the embodiment, as shown in the lower graph (power-time graph) of Fig. 14, the power is gradually reduced. At step S 20, since the temperature control of the term t2 has started, when eight second that is the set term control time passed, the process proceed to step S21. Then, the output is stopped and a coagulation or a coagulation dissection of the organism is finished. At steps S18 and S 19, if the foot switch 6 is turned off, the output is stopped.

In the above output control described with reference to Fig. 14, the condition for switching the controls from the term t1 to the term t2 is the temperature. Now, with respect to the condition for switching the control from the term t1 to the term t2, an output state in a case that a term control time functions is described with reference to Fig. 20.

Here, a constant power set value of the term t1 is set to a set value W2 [W], and the other set values are set similarly to the cases of Figs. 11 to 13. The set value W2 [W] is smaller than the set value W1 [W].

In response to the start of the output, as shown in a lower graph (power-time graph) of Fig. 20, first, at step S 12, the constant power control that is selected for the term t1 is performed in response to an application of the set value W2 [W]. Simultaneously with the output, as show in an upper graph (temperature - time graph) of Fig. 20, the temperature of the heating elements 21 is increased. At a time point when the term t1 control time, that is, four seconds, has passed, even if the temperature has not reached the limit temperature of the term t1 Temp 2 [°C], the output control is switched to the constant temperature control of the term t2 (steps S15 and S16). Then, the output is controlled such that the temperature of the heating elements 21 is kept to be the Temp 1 [°C] (step S 17). In such a case, the power may be applied such that the Temp 1 [°C] is kept. In the embodiment, as shown in the lower graph of Fig. 20, the power is gradually reduced. At step S 20, since the temperature control of the term t2 has started, when eight second that is the set term control time passed, at step S21, the output is stopped and a coagulation or a coagulation dissection of the organism is finished.

Accordingly, the above-described switching of the controls is performed based on a condition which has arrived faster than another condition between the limit temperature or the term control time.

In the above-described example, the case that the output sequence has two steps, that is, two terms of the term 1t and the term t2 has been described. However, only the term t1 may be controlled, or, three terms of the term t1, the term t2, and the term t3 may be controlled.

In such a case, at step S3 shown in Fig. 15, when the first output sequence switch 84 is depressed, only the term t1 is set as the output sequence. Then, at step S5, as the control performed in the term t1, for example, the constant power control shown in Fig. 12 which is previously set at step S1A is performed with the set value W1 [W]. Further, at step S6, in a case that a time has reached a control switching time (for example, four seconds shown in Fig. 13) of the term t1, a temperature has reached a limit temperature (for example, Temp 2 [°C] shown in Fig. 13), or the foot switch 6 is turned off, the process proceeds to step S7. Then, the output is stopped.

At step S 9 shown in Fig. 15, if the third output sequence switch 86 is depressed, the output sequence is controlled to have the three terms of the term t1, the term t2, and the term t3. In such a case, the process proceeds to the flowchart of Fig. 17. From step S22 to step S32, similarly to the above-described process performed in step S11 to step S21 of Fig. 16, the process is performed according to the setting set at step S1A of Fig. 15.

At step S 31 of Fig. 17, in a case that a time has reached a control switching time of the term t2, or a temperature has reached a limit temperature, at step S32 and step 33 of Fig. 18, the output control is switched from the control of the term t2 to the control of the term t3. Further, although the process of the term t3 is performed in step S34 to step S37 of Fig. 18, according to the setting set at step S1A of Fig. 15, the process is performed similarly to the process performed from step S18 to step S21 of Fig. 16.

Further, at step S10 of Fig. 15, if the joule control switch 87 is depressed, the process proceeds to step S38 of Fig. 19. If the foot switch 6 is turned on, at step S39, an output according to the constant heat quantity control is started. At step S42, if a consumed heat quantity has reached a set value, at step S43, the output is stopped. At steps S 40 and S 41, if the foot switch 6 is turned off, the output is stopped.

With respect to the combinations of the control contents, in the embodiment, the constant power control is selected in the term t1 and the constant temperature control is selected in the term t2. However, the selections and combinations may be selected from any of all combinations which can be made using the constant current control, the constant voltage control, the constant power control, and the constant temperature control. For example, in the term t1, the "constant voltage control" may be selected, in the term t2, the "constant temperature control" may be selected, and in the term t3, the "constant current control" may be selected. Further, a same control may be combined, for example, in the term t1, the "constant temperature control" may be selected, and also in the term t2, the "constant temperature control" may be selected.

According to the embodiment, a plurality of output controls may be combined to enable output controls other than the constant temperature control to expand the range of options in treatment. For example, the organism coagulation ability can be improved and the organism dissection time can be reduced. More specifically, for example, in a state that a living organism is sufficiently coagulated, a dissection can be quickly performed.

### (Second Embodiment)

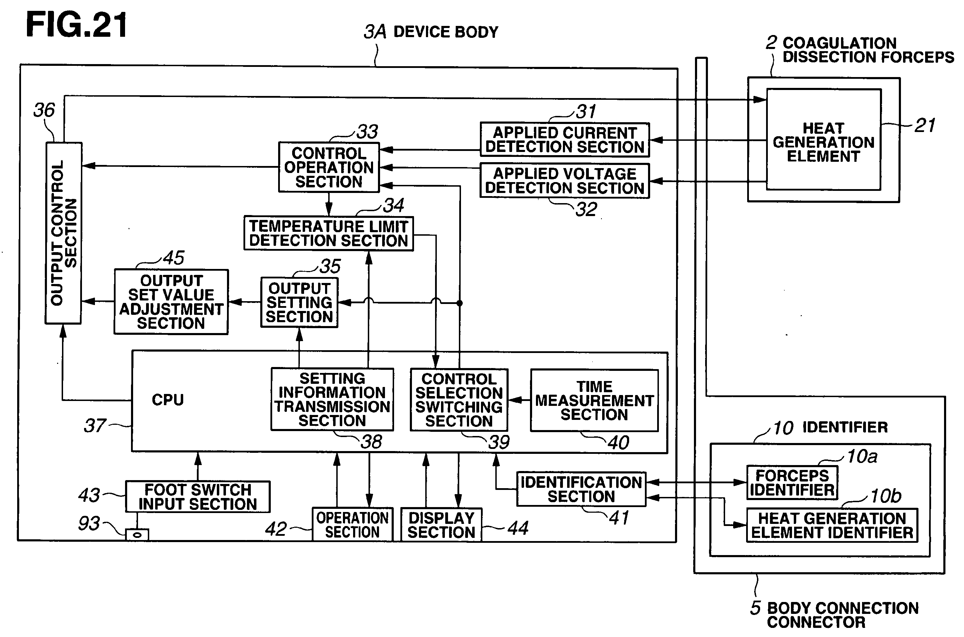

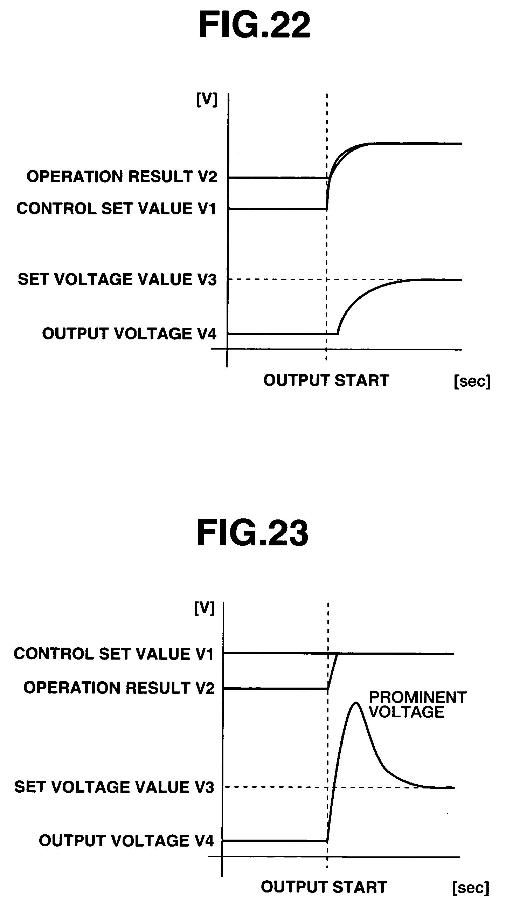

Figs. 21 and 22 relate to a second embodiment. Fig. 21 is a circuit block diagram of a heat treatment device. Fig. 22 is a graph illustrating a state of a control set value and an operation result, and a set voltage value and an output voltage value. Fig. 23 is a comparative explanatory diagram with Fig. 22 illustrating a prominent state of an output voltage due to a difference between a control set value and an operation result.

A device body 3A according to the embodiment differs from the device body 3 according to the first embodiment shown in Fig. 9. The device body 3A includes an output set value adjustment section 45 between the output setting section 35 and the output control section 36 as shown in Fig. 21. Other configurations are similar to those in the first embodiment. Accordingly, the same reference numerals are given to the similar configurations, their descriptions are omitted, and only the differences are described.

A control set value V1 shown in Fig. 22 represents a set value which is output from the output setting section 35. An operation result V2 shown in Fig. 22 represents a control operation result which is output from the output control operation section 33. A set voltage value V3 shown in Fig. 22 represents a set value which is actually output by the control by the output control section 36. An output voltage V4 shown in Fig. 22 represents an output value which is controlled by the output control section 36.

In the embodiment, an output control is performed such that the operation result V2 becomes the control set value V1 to set the output voltage V4 to be applied to the heating elements 21 to obtain a desired set voltage value V3.

In a heat control of the heat treatment device 1, by the output control section 36, an output is performed if the operation result V2 of the control operation section 33 is smaller than the control set value 1 of the output setting section 35. Then, the output control section 36, if the difference between the control set value V1 and the operation result V2 is large, increases the output value. On the other hand, if the difference between the control set value V1 and the operation result V2 is small, the output control section 36 decreases the output value. Further, the output control section 36 applies a current or a voltage such that the operation result V2 of the control operation section 33 and the control set value V1 of the output setting section 35 match each other.

Here, in the heat treatment device 1, as shown in Fig. 23, at a time of output start, if there is a large difference between the control set value V1 and the operation result V2, the output voltage V4 to be applied to the heating elements 21 becomes a maximum voltage (prominent voltage) which can be applied just after the output start. As a result, the control (temperature control) to the heating elements 21 may be unstable. Then, in the embodiment, as a mechanism for eliminating the prominent voltage at the time of the output start and stabilize the output control, an output set value adjustment section 45 is provided.

In the embodiment, as shown in Fig. 22, at a time the output setting section 35 starts an output of the control set value 1, set the value to be smaller than the operation result V2 which is outputted by the control operation section 33 or zero. Then, after the output is started, the control set value V1 is gradually smoothly increased to reduce as much as possible the "difference between the control set value V1 and the operation result V2", which is a condition the output voltage V4 generates, and generation of the prominent voltage is prevented. Then, the target set voltage value V3 is controlled to be maintained.

The control of "after the output is started, in a predetermined time period, the value is gradually smoothly increased to the predetermined control set value V1" is performed by the output set value adjustment section 45. In the embodiment, the control set value V1 is gradually smoothly increased by the output set value adjustment section 45. However, the method to increase the control set value V1 may be varied in a staircase pattern, that is, step by step. Further, the control set value V1 may be a predetermined function.

In the embodiment, by preventing the prominent voltage, the control can be stabilized.

### (Third Embodiment)

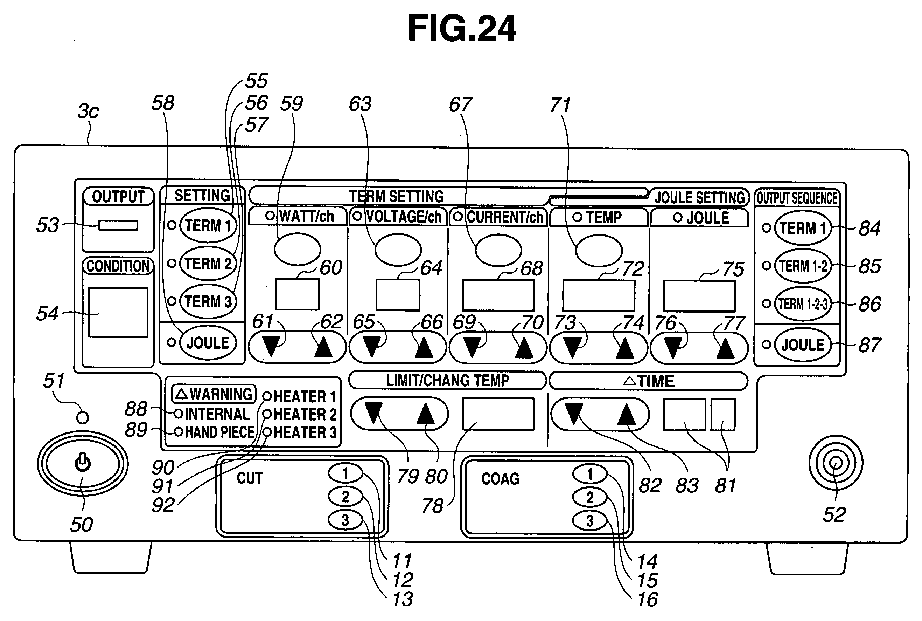

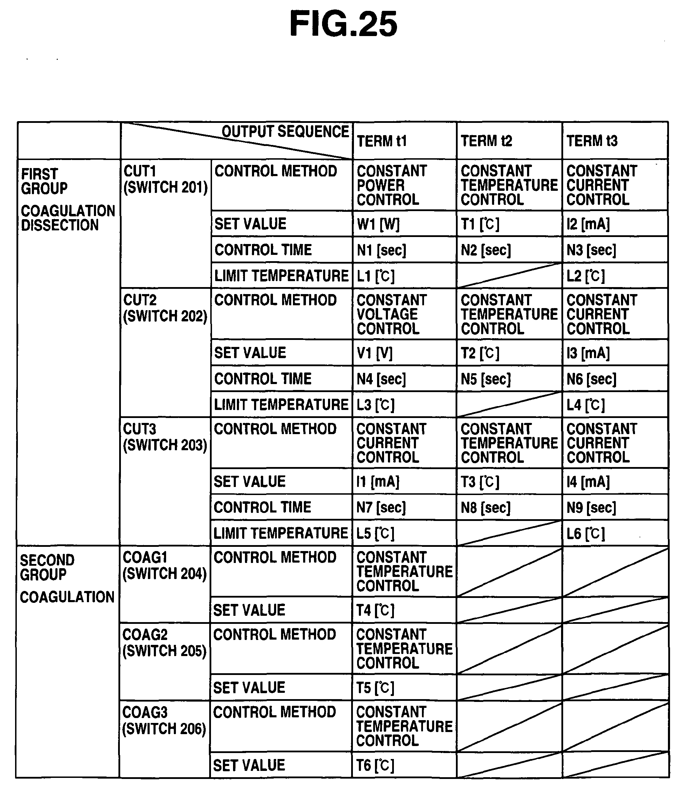

Figs. 24 and 25 relate to a third embodiment. Fig. 24 is an elevational view illustrating a device body viewed from a front panel side of the device body. Fig. 25 is a view illustrating output control combinations in a coagulation dissection setting of a heat treatment device and output control combinations in a coagulation setting of a heat treatment device.

The device body 3 shown in Fig. 24 has, on the front panel 3c, in addition to the configuration of the front panel 3a shown in Fig. 2 according to the first embodiment, the coagulation dissection output selection switches 11 to 13 and the coagulation output selection switches 14 to 16, which constitutes the operation section 42. To configurations similar to those in the first embodiment, same reference numerals are given and descriptions thereof are omitted, and only the differences are described. The switches 11 to 16 and the setting information transmission section 38 constitute combination control selection instruction means.

In the first embodiment, "output sequences, output control contents and set values of respective terms, and control switching conditions" can be arbitrarily set. However, in the embodiment, in addition to the arbitrary setting, the controls can be performed according to a previously set content.

As shown in Fig. 25, in items of coagulation dissection to be a first group, appropriate conditions to perform coagulation dissection, "output sequences, output control contents and set values of respective terms, and control switching conditions" are previously set. If any of the coagulation dissection output selection switches 11 to 13 is selected and depressed, output settings appropriate for the coagulation dissection output according to "output sequences, output control contents and set values of respective terms, and control switching conditions" can be set.

For example, if the coagulation dissection output selection switch 11 is depressed, the output sequence is set to three terms, that is, the term t1, the term t2, and the term t3. The control contents and set values are set, in the term t1, by the constant power control, the set value W1 [W], in the term t2, by the constant temperature control, the set value T1 [°C], and in the term t3, by the constant current control, the set value I2 [mA]. Further, the control switching condition to transfer from the term t1 to the term t2 is set according to a control time which is a predetermined time, that is, in a case that a term period reaches N1 [sec] or a limit temperature, which is a predetermined temperature, reaches L1 [°C]. The control switching condition to transfer from the term t2 to the term t3 is set according to a control time, that is, in a case that a term period reaches N2 [sec]. The control switching condition to finish the term t3 is set according to a control time, that is, in a case that a term period reaches N3 [sec] or a limit temperature reaches L2 [°C].

As shown in Fig. 25, in items of coagulation to be a second group, appropriate conditions to perform only the coagulation, "output sequences, output control contents and set values of respective terms, and control switching conditions" are previously set. If any of the coagulation output selection switches 14 to 16 is selected and depressed, output settings appropriate for the coagulation output according to the "output sequences, output control contents and set values of respective terms, and control switching conditions" can be set.

For example, if the coagulation output selection switch 14 is depressed, the output sequence is set to only the term t1. The control contents and set values are set, in the term t1, by the constant temperature control, the set value T4 [°C]. The control switching condition has not been set.

As described above, in the embodiment, the control combination settings for the coagulation dissection and the control setting for the coagulation are previously set. Then, the coagulation dissection control is selected using the coagulation dissection output selection switches 11 to 13, or, the coagulation control is selected using the coagulation output selection switches 14 to 16 to achieve the appropriate coagulation dissection or the coagulation.

It is to be understood that the combinations of output limitations in respective terms shown in Fig. 26 are one example, and combinations other than the above-described combinations may be used.

In the embodiment, it is not necessary to previously perform the settings. Accordingly, optimal output controls for the coagulation dissection or the coagulation can be performed much more easily respectively as compared with the case of the first embodiment. Other effects are similar to those in the first embodiment.

It is to be noted that the present invention is not limited to the above-described embodiments, but various modifications can be made without departing from the scope of the invention.

## Claims

1. A heat treatment device comprising:
coagulation/dissection means (2) having a treatment portion (7) adapted to treat a living organism and heating means (21) adapted to generate a heat to be applied to the organism provided in the treatment portion;
applied current detection means (31) adapted to detect a current value applied to the heating means;
applied voltage detection means (32) adapted to detect a voltage value applied to the heating means;
operation means (33) adapted to selectively perform operation of one of a current, a voltage, a power, and a temperature at the heating means using respective detection results of the applied current detection means and the applied voltage detection means;
output setting means (59, 61, 62, 63, 65, 66, 67, 69, 70, 71, 73, 74) adapted for selectively setting, as a control content of an output sequence for the heating means, one of a constant current control, a constant voltage control, a constant power control, and a constant temperature control for each of two or more terms of the control content;
an output sequence specifying section (84, 85, 86, 87) adapted to specify the output sequence;
a transition condition setting section (78, 79, 80, 81, 82, 83) adapted to set a temperature or a time period as a transition condition between the two or more terms;
output control means (36) adapted to control an output to the heating means based on the output sequence specified by the output sequence specifying section, an operation result by the operation means, and the temperature or the time period set by the transition condition setting section; and
control selection means (39) adapted to select one of the constant current control, the constant voltage control, the constant power control, and the constant temperature control as the control of the output control means according to the control content.

2. The heat treatment device according to claim 1, further comprising control selection instruction means adapted to instruct the selection of at least one or more controls out of the constant current control, the constant voltage control, the constant power control, and the constant temperature control in the control selection means.

3. The heat treatment device according to claim 1, wherein the heating means comprises one or a plurality of heating elements.

4. The heat treatment device according to claim 1, further comprising output on/off means configured for controlling on/off of an output to the heating means.

5. The heat treatment device according to claim 1, wherein the control selection means is adapted to combine two or more controls out of the current control, the voltage control, the constant power control, and the constant temperature control for a coagulation dissection treatment or a coagulation treatment, and is adpated to select the combination.

6. The heat treatment device according to claim 5, further comprising combination control selection instruction means configured for instructing the selection of the combination.

7. The heat treatment device according to claim 1, wherein the output control means is adapted to stop the output to the heating means in a case that the heat quantity applied to the heating means reaches a predetermined heat quantity.

8. The heat treatment device according to claim 7, further comprising heat quantity setting means for configured instructing to set the predetermined heat quantity.

9. The heat treatment device according to claim 1, further comprising:
time measurement means adapted to measure a time and determining a predetermined time; and
predetermined temperature detection means adapted to detect whether the heating means has reached a predetermined temperature or not;
wherein, the predetermined condition in the control selection means is at least one of the predetermined time determined by the time measurement means and the predetermined temperature detected by the predetermined temperature detection means.

10. The heat treatment device according to claim 9, further comprising temperature setting means configured for instructing the setting of the predetermined temperature at the predetermined temperature detection means.

11. The heat treatment device according to claim 9, further comprising time setting means configured for instructing the setting of the predetermined time at the time measurement means.

12. The heat treatment device according to any one of claims 1 to 11, further comprising output set value adjustment means configured for adjusting a difference between the operated value operated by the operation means and the value to be set by the output setting means to prevent a prominent voltage at a time of an output start to the heating means.

13. The heat treatment device according to claim 12, wherein the output set value adjustment means is adapted to perform a control, with respect to the output control means, at the time of the output start, to set an initial value to be set by the output setting means to a value smaller than the operated value of the operation means, and to increase the set value to a predetermined set value after a predetermined time has passed.

## Patentansprüche

1. Wärmebehandlungseinrichtung, die umfasst:
ein Koagulations/Seziermittel (2) mit einem Behandlungsabschnitt (7), der dazu eingerichtet ist, einen lebenden Organismus zu behandeln, und einem Heizmittel (21), das dazu eingerichtet ist, Wärme zu erzeugen, die dazu vorgesehen ist, auf den in dem Behandlungsabschnitt vorhandenen Organismus aufgebracht zu werden;
ein Mittel (31) zur Erfassung eines angelegten Stroms, das dazu eingerichtet ist, einen an das Heizmittel angelegten Stromwert zu erfassen;
ein Mittel (32) zur Erfassung einer angelegten Spannung, das dazu eingerichtet ist, einen an das Heizmittel angelegten Spannungswert zu erfassen;
ein Operationsmittel (33), das dazu eingerichtet ist, unter Verwendung jeweiliger Erfassungsergebnisse des Mittels zur Erfassung eines angelegten Stroms und des Mittels zur Erfassung einer angelegten Spannung selektiv eine Stromoperation, eine Spannungsoperation, eine Leistungsoperation und eine Temperaturoperation an dem Heizmittel auszuführen;
ein Ausgabefestlegungsmittel (59, 61, 62, 63, 65, 66, 67, 69, 70, 71, 73, 74), das dazu eingerichtet ist, als einen Steuerungsinhalt einer Ausgabesequenz für das Heizmittel selektiv eine konstante Stromsteuerung, eine konstante Spannungssteuerung, eine konstante Leistungssteuerung oder eine konstante Temperatursteuerung für jeden von zwei oder mehr Termen des Steuerungsinhalts festzulegen;
einen Ausgabesequenzspezifikationsabschnitt (84, 85, 86, 87), der dazu eingerichtet ist, die Ausgabesequenz zu spezifizieren;
einen Übergangsbedingungsfestlegungsabschnitt (78, 79, 80, 81, 82, 83), der dazu eingerichtet ist, eine Temperatur oder eine Zeitspanne als eine Übergangsbedingung zwischen den zwei oder mehr Termen festzulegen;
ein Ausgabesteuerungsmittel (36), das dazu eingerichtet ist, eine Ausgabe an das Heizmittel auf der Basis der von dem Ausgabesequenzspezifikationsabschnitt spezifizierten Ausgabesequenz, einem Operationsergebnis durch das Operationsmittel und der Temperatur oder der Zeitspanne, die durch den
Übergangsbedingungsfestlegungsabschnitt festgelegt worden sind, zu steuern; und
ein Steuerungsauswahlmittel (39), das dazu eingerichtet ist, die konstante Stromsteuerung, die konstante Spannungssteuerung, die konstante Leistungssteuerung oder die konstante Temperatursteuerung als die Steuerung des Ausgabesteuerungsmittels gemäß dem Steuerungsinhalt auszuwählen.

2. Wärmebehandlungseinrichtung nach Anspruch 1, die ferner ein Steuerungsauswahlbefehlsmittel umfasst, das dazu eingerichtet ist, die Auswahl mindestens einer oder mehrerer Steuerung(en) aus der konstanten Stromsteuerung, der konstanten Spannungssteuerung, der konstanten Leistungssteuerung und der konstanten Temperatursteuerung in dem Steuerungsauswahlmittel zu befehlen.

3. Wärmebehandlungseinrichtung nach Anspruch 1, bei der das Heizmittel ein Heizelement oder eine Mehrzahl von Heizelementen umfasst.

4. Wärmebehandlungseinrichtung nach Anspruch 1, die ferner ein Ausgabe-An/Aus-Mittel umfasst, das dazu eingerichtet ist, ein An/Aus einer Ausgabe an das Heizmittel zu steuern.

5. Wärmebehandlungseinrichtung nach Anspruch 1, bei der das Steuerungsauswahlmittel dazu eingerichtet ist, zwei oder mehr Steuerungen der Stromsteuerung, der Spannungssteuerung, der konstanten Leistungssteuerung und der konstanten Temperatursteuerung für eine Koagulationssektionsbehandlung oder eine Koagulationsbehandlung zu kombinieren, und dazu eingerichtet ist, die Kombination auszuwählen.

6. Wärmebehandlungseinrichtung nach Anspruch 5, die ferner ein Kombinationssteuerungsauswahlbefehlsmittel umfasst, das dazu eingerichtet ist, die Auswahl der Kombination zu befehlen.

7. Wärmebehandlungseinrichtung nach Anspruch 1, bei der das Ausgabesteuerungsmittel dazu eingerichtet ist, die Ausgabe an das Heizmittel zu stoppen, wenn die auf das Heizmittel aufgebrachte Wärmemenge eine vorbestimmte Wärmemenge erreicht.

8. Wärmebehandlungseinrichtung nach Anspruch 7, die ferner ein Wärmemengenfestlegungsmittel umfasst, das dazu eingerichtet ist, das Festlegen der vorbestimmten Wärmemenge zu befehlen.

9. Wärmebehandlungseinrichtung nach Anspruch 1, die ferner umfasst:
ein Zeitmessmittel, das dazu eingerichtet ist, eine Zeit zu messen und eine vorbestimmte Zeit zu bestimmen; und
ein Mittel zur Erfassung einer vorbestimmten Temperatur, das dazu eingerichtet ist, zu erfassen, ob das Heizmittel eine vorbestimmte Temperatur erreicht hat oder nicht;
wobei die vorbestimmte Bedingung in dem Steuerungsauswahlmittel die durch das Zeitmessmittel bestimmte vorbestimmte Zeit und/oder die von dem Mittel zur Erfassung der vorbestimmten Temperatur erfasste vorbestimmte Temperatur ist/sind.

10. Wärmebehandlungseinrichtung nach Anspruch 9, die ferner ein Temperaturfestlegungsmittel umfasst, das dazu eingerichtet ist, die Festlegung der vorbestimmten Temperatur an dem Mittel zur Erfassung der vorbestimmten Temperatur zu befehlen.

11. Wärmebehandlungseinrichtung nach Anspruch 9, die ferner ein Zeitfestlegungsmittel umfasst, das dazu eingerichtet ist, die Festlegung der vorbestimmten Zeit an dem Zeitmessmittel zu befehlen.

12. Wärmebehandlungseinrichtung nach einem der Ansprüche 1 bis 11, die ferner ein Mittel zur Einstellung eines ausgegebenen festgelegten Werts umfasst, das dazu eingerichtet ist, eine Differenz zwischen dem durch das Operationsmittel betätigten Operationswert und dem Wert einzustellen, der durch das Ausgabefestlegungsmittel festzulegen ist, um eine markante Spannung zu einem Zeitpunkt eines Ausgabestarts an das Heizmittel zu verhindern.

13. Wärmebehandlungseinrichtung nach Anspruch 12, bei der das Mittel zur Einstellung eines ausgegebenen festgelegten Werts dazu eingerichtet ist, zum Zeitpunkt des Ausgabestarts bezüglich des Ausgabesteuerungsmittel eine Steuerung auszuführen, um einen durch das Ausgabefestlegungsmittel festzulegenden Startwert auf einen Wert festzulegen, der geringer ist als der Operationswert des Operationsmittels und um den festgelegten Wert nach dem Ablauf einer vorbestimmten Zeit auf einen vorbestimmten festgelegten Wert zu erhöhen.

## Revendications

1. Dispositif de traitement thermique comprenant :
un moyen (2) de coagulation/dissection ayant une partie (7) de traitement adaptée à traiter un organisme vivant et un moyen (21) de chauffage adapté à générer une chaleur destinée à être appliquée à l'organisme prévu dans la partie de traitement ;
un moyen (31) de détection de courant appliqué adapté à détecter une valeur de courant appliqué au moyen de chauffage ;
un moyen (32) de détection de tension appliquée adapté à détecter une valeur de tension appliquée au moyen de chauffage ;
un moyen (33) d'opération adapté à sélectivement exécuter une opération d'un parmi un courant, une tension, une puissance et une température au niveau du moyen de chauffage en utilisant des résultats de détection respectifs du moyen de détection de courant appliqué et du moyen de détection de tension appliquée ;
un moyen (59, 61, 62, 63, 65, 66, 67, 69, 70, 71, 73, 74) de fixation de sortie adapté à sélectivement fixer, comme un contenu de commande d'une séquence de sortie pour le moyen de chauffage, une parmi une commande de courant constant, une commande de tension constante, une commande de puissance constante et une commande de température constante pour chacun de deux termes ou plus du contenu de commande ;
une section (84, 85, 86, 87) de spécification de séquence de sortie adaptée à spécifier la séquence de sortie ;
une section (78, 79, 80, 81, 82, 83) de fixation de condition de transition adaptée à fixer une température ou une période de temps comme une condition de transition entre les deux termes ou plus ;
un moyen (36) de commande de sortie adapté à commander une sortie du moyen de chauffage sur la base de la séquence de sortie spécifiée par la section de spécification de séquence de sortie, d'un résultat d'opération par le moyen d'opération, et de la température ou de la période de temps déterminée par la section de détermination de condition de transition ; et
un moyen (39) de sélection de commande adapté à sélectionner une parmi la commande de courant constant, la commande de tension constante, la commande de puissance constante, et la commande de température constante comme la commande du moyen de commande de sortie en fonction du contenu de commande.

2. Dispositif de traitement thermique selon la revendication 1, comprenant en outre un moyen d'instruction de sélection de commande adapté à indiquer la sélection d'au moins une commande ou plus parmi la commande de courant constant, la commande de tension constante, la commande de puissance constante et la commande de température constante dans le moyen de sélection de commande.

3. Dispositif de traitement thermique selon la revendication 1, dans lequel le moyen de chauffage comprend un ou une pluralité d'éléments de chauffage.

4. Dispositif de traitement thermique selon la revendication 1, comprenant en outre un moyen d'activation/désactivation de sortie configuré pour commander une activation/désactivation d'une sortie vers les éléments de chauffage.

5. Dispositif de traitement thermique selon la revendication 1, dans lequel le moyen de sélection de commande est adapté à combiner deux commandes ou plus parmi la commande de courant, la commande de tension, la commande de puissance constante et la commande de température constante pour un traitement de coagulation/dissection ou un traitement de coagulation, et est adapté à sélectionner la combinaison.

6. Dispositif de traitement thermique selon la revendication 5, comprenant en outre un moyen d'instruction de sélection de commandes en combinaison configuré pour indiquer la sélection de la combinaison.

7. Dispositif de traitement thermique selon la revendication 1, dans lequel le moyen de commande de sortie est adapté à stopper la sortie vers le moyen de chauffage dans un cas où la quantité de chaleur appliquée au moyen de chauffage atteint une quantité de chaleur prédéterminée.

8. Dispositif de traitement thermique selon la revendication 7, comprenant en outre un moyen de fixation de quantité de chaleur configuré pour indiquer de fixer la quantité de chaleur prédéterminée.

9. Dispositif de traitement thermique selon la revendication 1, comprenant en outre :
un moyen de mesure de temps adapté à mesurer un temps et déterminant un temps prédéterminé ; et
un moyen de détection de température prédéterminée adapté à détecter si le moyen de chauffage a atteint une température prédéterminée ou non ;
dans lequel la condition prédéterminée dans le moyen de sélection de commande est au moins un parmi le temps prédéterminé déterminé par le moyen de mesure de temps et la température prédéterminée détectée par le moyen de détection de température prédéterminée.

10. Dispositif de traitement thermique selon la revendication 9, comprenant en outre un moyen de fixation de température configuré pour indiquer la fixation de la température prédéterminée au moyen de détection de température prédéterminée.

11. Dispositif de traitement thermique selon la revendication 9, comprenant en outre un moyen de fixation de temps configuré pour indiquer la fixation du temps prédéterminé au moyen de mesure de temps.

12. Dispositif de traitement thermique selon l'une quelconque des revendications 1 à 11, comprenant en outre un moyen d'ajustement de valeur fixée de sortie configuré pour ajuster une différence entre la valeur opérée par le moyen d'opération et la valeur devant être fixée par le moyen de fixation de sortie pour prévenir une tension importante à un moment d'un début de sortie vers le moyen de chauffage.

13. Dispositif de traitement thermique selon la revendication 12, dans lequel le moyen d'ajustement de valeur fixée de sortie est adapté à exécuter une commande, par rapport au moyen de commande de sortie, au moment du début de sortie, à fixer une valeur initiale devant être fixée par le moyen de fixation de sortie à une valeur plus petite que la valeur opérée du moyen d'opération, et à augmenter la valeur fixée à une valeur fixée prédéterminée après qu'un temps prédéterminé s'est écoulé.
